# EUROPÄISCHE PATENTSCHRIFT

(11) **EP 1 283 011 B1**
(45) Veröffentlichungstag und Bekanntmachung des Hinweises auf die Patenterteilung: **02.04.2008**
(21) Anmeldenummer: 01118972.7
(22) Anmeldetag: 06.08.2001
(51) Int. Cl.: A21D 2/18, A21D 8/04, C12P 19/04, C12M 1/20

(54) **Verfahren zur Herstellung von prebiotischen Sauerteigen/Vorteigen**
Process for the production of prebiotic sourdoughs
Procédé pour la préparation d'un levain de panification prébiotique

(43) Veröffentlichungstag der Anmeldung: 12.02.2003
(73) Patentinhaber: Ernst Böcker GmbH & Co. KG, 32427 Minden (DE)
(72) Erfinder: Stolz, Peter, Dr., 32457 Porta Westfalica (DE); Vogel, Rudi, 85141 Kirchdorf-Wippenhausen (DE); Korakli, Maher, 85356 Freising (DE); Gänzle, Michael, 85354 Freising (DE); Hammes, Walter Peter, 70794 Filderstadt (DE); Brandt, Markus, 70794 Filderstadt (DE)
(74) Vertreter: Schneiders, Josef

(56) Entgegenhaltungen:
- EP-A- 0 790 003
- EP-A- 1 110 458
- US-A- 4 243 687
- US-A- 4 423 079
- US-A- 5 834 043
- M. KORAKLI: "Production of exopolysaccharide in wheat and rye sourdoughs by Lactobacillus sanfranciscensis" EXOPOLYSACCHARIDES FROM LACTIC ACID BACTERIA ; SYMPOSIUM, [Online] 17. Mai 2001 (2001-05-17), Seite 2 XP002188904 Brussels Belgium Gefunden im Internet: <URL:http://imol.vub.ac.be/IMDO/congres/fi nalprogram.html> [gefunden am 2002-01-31] -& KORAKLI M.: "sucrose metabolism and exopolysaccharide production in wheat and rye sourdoughs by Lactobacillus sanfranciscensis" JOURNAL OF AGRICULTURAL AND FOOD CHEMISTRY, Bd. 49, Nr. 11, 2001, Seiten 5194-5200, XP002188905
- KORAKLI M ET AL: "PRODUCTION OF MANNITOL BY LACTOBACILLUS SANFRANCISCENSIS" ADVANCED IN FOOD SCIENCES, TECHNISCHE UNIVERSITAET MUENCHEN, MUENCHEN, DE, Bd. 22, Nr. 1/2, 2000, Seiten 1-4, XP001016281 ISSN: 1431-7737
- FIGUEROA C.: "Lactic acid bacteria of the sour cassava starch fermentation" LETTERS IN APPLIED MICROBIOLOGY, Bd. 21, 1995, Seiten 126-130, XP008000236

## Beschreibung

Die Erfindung betrifft ein Verfahren zur Herstellung von prebiotischen Sauerteigen/Vorteigen aus mindestens einem Getreidemahlgut und Wasser unter Zusatz von mindestens einer Starterkultur, ggf. unter Weiterführung des Sauerteiges/Vorteiges unter fortlaufender Säuerung mittels Zugabe von Mehl und Wasser.

Die Erfindung betrifft ferner polyfructosanebildende Milchsäurebakterien der Art Lactobacillus sanfranciscensis, welche sich für die Durchführung des Verfahrens in besonderem Maße eignen.

In Ländern mit hohem Roggenverzehr dient die Verwendung von Roggensauerteigen zur Säuerung, zur Geschmacks- und Aromasteigerung sowie zur Lockerung des Roggen-, Roggengemisches und Weizenmisch-Brotteiges. Bei der Herstellung von Weizenbroten besteht keine technologische Notwendigkeit zur Säuerung des Weizenbrotteiges; traditionell werden aber häufig Vorstufen in Form von Weizenvorteigen oder milden Weizensauerteigen geführt, die der Geschmacks- und Aromaverbesserung sowie der Verbesserung der Frischhaltung und des Schimmelschutzes dienen.

Sauerteig- und Vorteig-Fermentationen können spontan eingeleitet werden. Spontan eingeleitete Fermentationsprozesse unterliegen jedoch einem erhöhten mikrobiologischen Risiko, da es häufig zu Fehlfermentationen kommt, die zu qualitativ minderwertiger Brotqualität führen können.

Weizenvorteige werden in der Regel mit Backhefen beimpft. Backhefen sind jedoch häufig mit Milchsäurebakterien verunreinigt, wodurch es zu einer unkontrollierten Säurebildung bei Hefevorteig-Führungen kommen kann. Die in Backhefen gefundene Milchsäurebakterien sind in der Regel nicht oder nur mangelhaft an das Habitat Getreide bzw. Teig angepasst. Ferner unterliegt deren Keimzahl erheblichen Schwankungen, weshalb eine reproduzierbare Vorteigqualität des Vorteiges nicht gewährleistet werden kann.

Sauerteig- und Vorteig-Fermentationen sind nicht auf die bloße Säuerung und Gasbildung im Teig beschränkt. Auch Quellungsvorgänge und die Bildung von Aromapräkursoren aus dem Eiweiß-, Fett- und Kohlenhydratstoffwechsel der beteiligten Mikroorganismen sind von wertbestimmender Bedeutung für die Qualität der Backwaren. Die in Sauerteigen/Vorteigen gebildeten Aromapräkursoren werden später im Backprozess in Aromastoffe umgewandelt und tragen wesentlich zum vorteigtypischen Geschmack und Aroma der Weizengebäcke bei. Ein zusätzlicher positiver Effekt von gesäuerten Teigen gegenüber reinen Hefeteigen ist die Unterdrückung des Wachstums von Schimmelpilzen und fadenziehenden Bakterien, wodurch die Haltbarkeit und Lagerstabilität von gesäuerten Backwaren im Vergleich zu ungesäuerten bzw. nicht ausreichen gesäuerten Gebäcken deutlich verbessert ist.

Die Herstellung von Sauerteig- bzw. Vorteig-Startern kann wie folgt erfolgen:
(a) durch technologische "Reinkulturverfahren" unter sterilen Bedingungen in speziellen Nährmedien in Fermentern. Diese Reinkulturen sind einfach und reproduzierbar zu kultivieren, aber häufig durch die Kultivierung in künstlichen Nährmedien nicht mehr oder nur noch mangelhaft an die Bedingungen im Sauerteig bzw. Vorteig angepaßt. Daher sind sie in der Praxis unter unsterilen Bedingungen im Teig oft unsicher im Durchsetzungsvermögen.
(b) durch "natürliche Reinzucht" mittels wiederholter Rekultivierungszyklen im Teig. Mit Hilfe dieses Herstellungsverfahren lassen sich in der Regel besonders gut an die Milieubedingungen der jeweiligen Sauerteig-bzw. Vorteigfermentation angepaßte Starterkulturen herstellen.

Die Säuerung bei Sauerteigen und Vorteigen basiert auf dem Stoffwechsel von Milchsäurebakterien, im wesentlichen von Laktobazillen, die aufgrund ihrer Stoffwechseleigenschaften als homo- oder heterofermentativer Laktobazillen klassifiziert werden. Die Gasentwicklung beruht in den meisten Sauerteigen auf der Stoffwechselaktivität von heterofermentativen Laktobazillen und auf besonderer Sauerteighefen.

Die Bakterienflora von Sauerteigen besteht in der Regel aus erstens homofermentativen Laktobazillen, die Glucose fast ausschließlich zu Milchsäure verstoffwechseln, und zweitens aus heterofermentativen Laktobazillen, die Glucose bzw. Maltose aufgrund einer anderen Enzymausstattung zu Milchsäure, CO₂ und Ethanol oder Essigsäure vergären.

In herkömmlichen Sauerteigen werden in der Regel Hefen, die in erster Linie für die Teiglockerung verantwortlich sind, und eine kompliziert zusammengesetzte Bakterienflora, die in erster Linie für die Säurebildung verantwortlich sind, vermehrt.

Zur Herstellung von Sauerteigen werden ferner verschiedene Führungstechniken herangezogen, die sich vor allem im Zeit- und Arbeitsaufwand unterscheiden. Man unterscheidet zwischen direkten und indirekten Führungsweisen, wobei bei letzterer noch zwischen einstufigen und mehrstufigen Führungsweisen unterschieden werden kann.

Die mehrstufige Führung berücksichtigt die unterschiedlichen Anforderungen, die von Hefen und Bakterien an Temperatur und Feuchtigkeit gestellt werden. Meist besteht die mehrstufige Führung aus Anstellgut, Anfrischsauer, Grundsauer und Vollsauer. Bei einstufiger Führung wird auf die Entwicklung der Hefen keine Rücksicht genommen. Hierbei wird nur eine Sauerstufe mit relativ viel Anstellgut angesetzt.

Bei Vorteigführungen wird dem Teig traditionell eine Backhefe beigemengt. Backhefen sind jedoch häufig mit Milchsäurebakterien verunreinigt, wodurch es hauptsächlich bei längeren Hefevorteig-Führungen zu einer unkontrollierten Säuerung kommen kann. Die in Backhefen gefundene Milchsäurebakterien sind in der Regel nicht oder nur mangelhaft an das Habitat Getreide bzw. Teig angepaßt. Desweiteren unterliegt deren Keimzahl erheblichen Schwankungen, weshalb eine reproduzierbare Vorteigqualität nicht gewährleistet werden kann.

Bei der Herstellung von Backwaren werden häufig Backmittel (Backhilfsmittel) eingesetzt. Ihre Aufgabe besteht darin, die Herstellung von Backwaren zu erleichtern oder zu vereinfachen, die wechselnden Verarbeitungseigenschaften der Rohstoffe auszugleichen und die Qualität der Backwaren positiv zu beeinflussen.

Es gibt eine Reihe von Backhilfsmitteln, die sich auf die Teigrheologie und/oder das Backergebnis auswirken. Zu nennen sind hier z. B. Guar, native Kleberproteine, enzymaktives Malz- oder Sojamehl, Cystein und vieles mehr. Durch Zugabe geringer Mengen an bestimmten Backhilfsmitteln (z. B. Guar, native Kleberproteine, enzymaktives Malz- oder Sojamehl) wird z. B. kleberschwaches Mahlgut im Hinblick auf die Herstellung von Brot und Brötchen verbessert, da der Teig an Trockenheit, Dehnungswiderstand, Knettoleranz und Gärstabilität zunimmt. Außerdem steigt das Gebäckvolumen und es verbessert sich die Krumenstruktur. Andere Backhilfsmittel (z. B. Cystein) wiederum bewirken eine Erweichung bzw. Plastifizierung des Teiges, so daß sich mit nur geringem Energieaufwand der Teig gut verformen läßt.

Einerseits ist der Einsatz von Backhilfsmitteln in der Backwarenindustrie oder größeren Bäckereien nicht mehr wegzudenken, da eine gleichbleibende Qualität der Backware von Verbrauchern erwartet wird. Andererseits jedoch wird die Zugabe von "künstlichen" Backhilfsmitteln, wie z. B. Enzymen, zur Beeinflussung der Backeigenschaften zunehmend skeptischer betrachtet, da sich die Einstellung der Konsumenten zu artfremden Zusatzstoffen, auch aufgrund von Lebensmittelskandalen, geändert hat.

Verbraucher verhalten sich ernährungs- und gesundheitsbewußt, auch was die Wahl ihrer Nahrungsmittel anbetrifft. Aus diesen Gründen werden an Lebensmittel hohe Anforderungen gestellt. Sie sollen bei gleichbleibend hoher Qualität gleichzeitig eine gesundheitsfördernde Funktion aufweisen und des weiteren auf naturbelassener Rohware basierend hergestellt sein. Aus ernährungsphysiologischem und "funktionalem" Aspekt werden daher Probiotika und Prebiotika eingesetzt.

Probiotika sind Bakterien, meist Bifidusbakterien oder Laktobazillen, die in ausreichender Menge lebend in den Dickdarm gelangen und dort für eine ausgewogene Darmflora sorgen. Probiotische Produkten mit derart angereicherten Bakterien haben eine positive Wirkung auf das Immunsystem und das allgemeine Wohlbefinden. Derart zugeführte Probiotika müssen in regelmäßigen Abständen dem Körper zugefügt werden, da die Bakterien entweder absterben oder ausgeschieden werden. Für die Förderung und Erhaltung dieser Mikroflora im Dickdarm werden daher dem Lebensmittel neuerdings Prebiotika zugefügt.

Prebiotisch wirksame Nahrungsbestandteile (sogenannte Ballaststoffe), sind für den Menschen unverdaulich und gelangen dadurch in untere Darmabschnitte, wo sie den erwünschten probiotischen Bakterien als Nahrung dienen und damit die Zusammensetzung der Darmflora günstig beeinflussen. Letztendlich wird durch eine "gesunde Darmflora" die Gesundheit des Menschen auf vielfältige Weise positiv beeinflusst.

Probiotika, also lebende Bakterien, spielen bei Backwaren keine besondere Rolle, da diese durch die hohe Backtemperatur abgetötet werden. Prebiotika werden zur Zeit in Molkereiprodukten und seit kurzem auch in Wurstprodukten eingesetzt. Als Prebiotika in Backwaren werden bisher Inuline und FOS (Fructooligosaccharide) als deklarationspflichtige Zusatzstoffe eingesetzt.

Hinsichtlich dieser Anforderungen an Lebensmitteln, insbesondere Backwaren, besteht die Aufgabe der Erfindung darin, ein Verfahren zur Herstellung von prebiotischem Sauerteig/Vorteig zur Verfügung zu stellen, welches die Verarbeitungseigenschaften des Mahlgutes unter Verminderung des Einsatzes von Backhilfsmitteln, insbesondere durch Erhöhung der Wasserbindung, optimiert, sowie eine prebiotische Wirkung der daraus hergestellten Backwaren mit sich bringt. Es wäre auch wünschenswert, ein Verfahren bereitzustellen, mit dem die "in situ" Bildung prebiotischer Substanzen im Teig selbst ermöglichen.

Zur Lösung dieser Aufgabe schlägt die Erfindung, ausgehend von einem Verfahren der eingangs genannten Art vor, daß das Anstellgut Exopolysaccharid bildende Mikroorganismen, bei denen es sich um Lactobacillus sanfranciscensis DSMZ 10613 oder DSMZ 14373 oder deren Mischung handelt, enthält. Durch die Zugabe dieser speziellen Mikroorganismen wird der Sauerteig bzw. Vorteig mit Exopolysacchariden angereichert, wobei es sich hierbei um extrazelluläre Polyfructosane handelt. Des Weiteren wird dem Getreidemahlgut Saccharose zugesetzt, wobei 0,2 - 30,0 Gew.-%, bevorzugt 1,0 - 20,0 Gew.%, besonders bevorzugt 5 - 15 Gew.-%, Saccharose bezogen auf das Mehl im Teig eingesetzt wird.

Bei einer Tagung in Brüssel vom 16. bis 19.05.2001 ist ein Verfahren zur Herstellung von Sauerteigen mit Lactobacillus sanfranciscensis unter Bildung von Polyfructosanen bekannt. Der Zusatz von Saccharosen und die in situ Bildung von prebiotischen wirksamen Substanzen ist ebenfalls offenbart worden.

Des Weiteren ist aus dem Journal of Agricultural and Food Chemistry 2001 (49) 11, 5194 - 5200 der Zusatz von Saccharose und die in situ Bildung von prebiotisch wirksamen Substanzen offenbart.

Mit dem erfindungsgemäßen Lactobacillus sanfranciscensis Stämmen werden die Teigrheologie im Gegensatz zum Stand der Technik deutlich verbessert.

Exopolysaccharide sind extrazelluläre mikrobielle Polysaccharide, deren Menge und chemische Struktur von der Art der eingesetzten Mikroorganismen und der Kohlenstoffquelle abhängig sind. Exopolysaccharide werden in Homo- und Hetero-Polysaccharide klassifiziert. Homo-Polysaccharide werden des weiteren unterteilt in Glucosetypen und Fructosetypen. Homo-Polysaccharide enthalten eine Art von Monosacchariden, z. B. besteht Dextran aus α-1,6-verknüpften Glucoseeinheiten und kann z. B. von dem Mikroorganismus Leuconostoc mesentroides gebildet werden. Auch β-2,6-verknüpfte Fructosane, welche von dem Mikroorganismus Streptococcus salivarius gebildet werden können, gehören zu dieser Gruppe.

Hetero-Polysaccharide enthalten dagegen verschiedene Arten von Monosacchariden. Hierzu zählen z. B. Xanthan, welches von dem Mikroorganismus Xanthomonas campestris gebildet werden kann.

Die Eigenschaften der Exopolysaccharide hängen von der Art und Anzahl der gebundenen Monosaccharide, von dem Grad der Verzweigung dieser Monosaccharid-Einheiten sowie von der Art der glykosidischen Bindungen ab.

Bei den erfindungsgemäßen Exopolysacchariden handelt es sich um Polyfructosane, die im wesentlichen Fructose enthalten.

Herkömmliche Polysaccharide auf pflanzlicher Basis, wie Stärke, Carrageen und Gummi arabicum, und mikrobielle Polysaccharide werden in der Lebensmittelproduktion eingesetzt, um physikalische Zustände herbeizuführen, die mit der Art und Menge der Wasserbindung in Beziehung stehen. Sie wirken auf die Konsistenz, Viskosität, Textur, Stabilität und Plastizität der mit ihnen zubereiteten Lebensmittel. Wichtige Verwendungsmöglichkeiten in Lebensmitteln sind Verdickungs-, Binde- und Quellmittel, Trubstabilisatoren, Schutzkolloide, Suspendiermittel, Schaumstabilisatoren, Kristallisations-Inhibitoren, Füllstoff, Emulsionsmittel und vieles mehr. Für viele der Verwendungszwecke werden auch Kombinationen der Polysaccharide herangezogen.

Es sind bereits extrazellulläre Polysaccharide beschrieben, die von Bakterien der Gattung Klebsiella von Stämmen der Art Klebsiella pneumoniae (US 4 298 691) und Klebsiella planticola (DE 3 445 302) erzeugt werden. Des weiteren beschreibt EP-A-0 790 003 dextranbildende Milchsäurebakterien und den Einsatz von Dextranen in Backwaren.

Die erfindungsgemäßen Exopolysaccharide werden von bestimmten Stämmen der Spezies Lb. sanfranciscensis produziert, die befähigt sind, ein Homo-Polysaccharid des Fructosetypes zu produzieren. Diesen sog. Polyfructosanen wird eine prebiotische Wirkung nachgesagt, da sie das Wachstum von Bifidobakterien im menschlichen Darm anregen und verstärken. Bestätigt wurde diese Erkenntnis in der wissenschaftlichen Arbeit von Korakli et al. (2002): Metabolism by bifidobacteria and lactic acid bacteria of polysaccharides from wheat and rye, and exopolysaccharides produced by Lactobacillus sanfranciscensis, J. Appl. Microbiol. 92: 958 - 965. Darin wird festgestellt, daß eine Reihe von Bifidobakterien die erfindungsgemäßen langkettigen Exopolysaccharide abbauen bzw. verstoffwechseln können. Auch die in-vitro-Studie von Dal Bello et al. (2001): In vitro study of Prebiotic Properties of Levantype Exopolysaccharides from Lactobacilli and Non-digestible Carbohydrates Using Denaturing Gradient Gel Electrophoresis. System. Appl. Microbiol. 24: 232 - 237, konnte zeigen, daß von den erfindungsgemäßen Mikroorganismen gebildetes Polyfructosan selektiv das Wachstum Bifidobakterien in Faeces stimuliert.

Die erfindungsgemäßen Laktobazillen wurden aus natürlichen Mischfloren isoliert und wurden besonders gut an die Bedingungen in Sauerteigen adaptiert. Diese Stämme zeichnen sich dadurch aus, dass Sie bei Zugabe von Saccharose zum Teig ein Exopolysaccharid bilden (Korakli et al. (2001): Sucrose metabolism and exopolysaccharide production in wheat and rye sourdoughs by Lactobacillus sanfranciscensis, J. Agric. Food Chem. 49: 5194-5200), das im wesentlichen aus Fructoseeinheiten besteht. Das von diesen Laktobazillen gebildete Exopolysaccharid (Polyfructosan) ist von besonderem Interesse, da Polyfructosane prebiotisch wirksam sind und im Darm vorhandene Bifidobakterien wirksam unterstützen.

Vorzugsweise werden als Kohlenstoffquelle Kohlenhydrate, insbesondere Saccharose, eingesetzt. Der Zusatz von Kohlenhydraten ist hierbei notwendig, da die polyfructosanbildenden Mikroorganismen die hochmolekularen Kohlenhydrate des Getreidemahlgutes (Stärke, Pentosane oder Fructosane) nicht zur Polyfructosanbildung verwerten können. Die geringe Menge an Saccharose in Roggen- (0,08 %) und Weizenmehl (0,06%) reichen nicht aus, um die Bildung von Exopolysacchariden in nennenswerten Mengen zu unterstützen. Zur Zellvermehrung wird dagegen die aus Stärkeabbau stammende Maltose benutzt.

Die Menge der einzusetzenden Kohlenhydrate (Saccharose) hängt von der Art und Menge des Mikroorganismus ab, liegt aber im allgemeinen zwischen 0,2 und 30,0 Gew.-%, insbesondere 1 - 20 Gew.%, bezogen auf das Mehl im Teig.

Es ist zweckmäßig, die Menge des Kohlenhydrats so zu wählen, daß einerseits genügend Exopolysaccharide im Sauerteig/Vorteig gebildet werden, so daß die technologische und prebiotische Wirkung in der Backware letztendlich vorhanden ist, andererseits aber der Süßungsgrad der Backware nicht beeinflußt wird. Eine bevorzugte Dosierung von Saccharose liegt bei 5 - 15 Gew.%, bezogen auf das Mehl.

Die erfindungsgemäßen Laktobazillen als Starterkulturen ermöglichen erstmals die "in situ Bildung" einer prebiotisch wirksamen Substanz im Sauerteig/Vorteig und damit die Herstellung von prebiotischen Backwaren, denen die prebiotisch wirksame Substanz nicht mehr zugesetzt werden muß. Außerdem werden durch die Bildung der erfindungsgemäßen Polyfructosane die rheologischen Eigenschaften der Teige positiv beeinflußt, insbesondere wird die Wasseraufnahmefähigkeit verbessert und die Klebrigkeit der Teige vermindert, und dadurch die Maschinengängigkeit der Teige verbessert.

Die mit diesen Kulturen hergestellten prebiotischen Backwaren zeichnen sich durch eine gute Frischhaltung und Texturbeschaffenheit aus, bei gleichzeitig sauerteigtypischem Aroma und Geschmack.

Untersuchungen haben gezeigt, daß die während der Sauerteig-Fermentation gebildeten Exopolysaccharide (Polyfructosane) einen großen Effekt auf die Teigrheologie haben. Der Zusatz von wasserbindenden Backhilfsmitteln kann somit reduziert werden oder entfallen.

Darüber hinaus werden Anforderungen an eine traditionelle und ökologische Backwarenherstellung erfüllt. Das Verfahren zeichnet sich dadurch aus, daß die teigrheologisch und prebiotisch wirksamen Polyfructosane durch die erfindungsgemäßen Laktobazillen während der Sauerteigfermentation selbst gebildet werden. Aus diesem Grund kann erstens der Einsatz von Backhilfsmittel zur Steigerung der Wasserbindung bzw. Frischhaltung reduziert werden oder sogar überflüssig werden und zweitens braucht man zur Herstellung von prebiotischen Backwaren keine prebiotisch wirksamen Nahrungsergänzungsmittel (z. B. Inuline und Fructooligosaccharide) dem Teig zusetzen, d.h. die Herstelllung von prebiotischen Backwaren kann ohne deklarierungspflichtige Zusatzstoffe erfolgen.

Da die erfindungsgemäßen Exopolysaccharide auf natürlichem Wege bzw. während der Sauerteig- bzw. Vorteigfermentation erzeugt werden, erfüllt die daraus hergestellte Backware die Verbrauchererwartungen an eine traditionell hergestellte Backware in vollem Maße.

Zur Herstellung von Polyfructosanen und/oder zur Anzucht der erfindungsgemäßen Bakterien wird zweckmäßig ein Nährmedium herangezogen, welches Saccharose enthält. Mit diesem speziellen erfindungsgemäß verwandten Nährmedium werden optimale Wachstumsbedingungen für die Polyfructosane-bildenden Laktobazillen erfüllt.

Ein erfindungsgemäßes künstliches Nährmedium besteht insbesondere aus Pepton (aus Casein), Hefeextrakt, Fleischextrakt, Dikaliumhydrogenphosphat, Kaliumdihydrogenphosphat, Cystein-HCI, Ammoniumchlorid, Saccharose sowie einer erfindungsgemäßen Stammlösung. Die Stammlösung enthält Magnesiumsulfate, Mangansulfate und einen Vitaminmix, welcher Biotin, Folsäure, Nikotinsäure, Pyridoxalphoshat, Thiamin, Riboflavin, Cobalamin und Panthothensäure aufweist.

Bei den erfindungsgemäßen Lactobacillen handelt es sich insbesondere um Lactobacillus sanfransiscensis DSMZ 10613 und Lactobacillus sanfransiscensis DSMZ 14373 als Einzelkultur oder als Mischkultur beider Stämme. Beide Stämme sind bei der DSMZ in Braunschweig nach den Bestimmungen des Budapester Vertrages hinterlegt. Sie können bei einer bestimmten Temperatur in einem erfindungsgemäßen Nährmedium unter kontrollierter Atmosphäre kultiviert werden. In den Produktionsmedien wird Saccharose als Kohlenstoffquelle für die Polyfructosanbildung verwandt. Die Temperatur liegt zweckmäßig bei 20 bis 35 °C, insbesondere bei etwa 25 bis 30 °C. Die Inkubationszeit beträgt 10 bis 72 Stunden. Die so kultivierten Mikroorganismen können als Impfgut für die Herstellung von polyfructosanhaltigen Sauerteigen/Vorteigen oder für die Erzeugung von Polyfructosanen im künstlichen Nährmedium dienen. Durch Zentrifugation können die Mikroorganismen geerntet werden, anschließend in einer physiologischen Kochsalzlösung resuspendiert und die gewaschene Zellmasse als Inokulat für die Bildung von Polyfructosanen verwendet werden.

Ein bevorzugtes typisches Ausführungsbeispiel der Erfindung zur Herstellung eines prebiotischen Sauerteiges/Vorteiges ist die Inokulation eines Teiges mit Lactobacillus sanfranciscensis DSMZ 10613 und Lactobacillus sanfranciscensis DSMZ 14373 als Einzelkultur und besonders bevorzugt als Mischkultur beider Stämme.

Zur Herstellung von prebiotischen SauerteigenNorteigen für die direkte Herstellung von prebiotischen Backwaren werden die erfindungsgemäßen Mikroorganismen als Starterkulturen zur "in situ Bildung" der Polyfructosane im Teig verwendet.

Als Getreidemahlprodukte können alle handelsüblichen Roggen- und Weizenmehle in Verbindung mit der empfohlenen Saccharosemenge zur Anwendung kommen. Bevorzugt wird bei Weizenmehlen ein Aschegehalt von 450 bis 1050 mg/100g, besonders bevorzugt 500 bis 630 mg/100g. Bei Roggenmehlen wird ein Aschegehalt von 997 bis 1800 mg/100g bevorzugt, besonders bevorzugt 1100 bis 1400 mg/100g.

Vorzugsweise können auch Getreidemahlprodukte aus Weizenkeimen, Dinkel, Gerste, Hafer und Reis zur Anwendung kommen.

Mit dem erfindungsgemäßen Sauerteig/Vorteig können prebiotsiche Backwaren, hergestellt werden, die einen hohen ernährungsphysiologischen Wert aufweisen.

Mit Hilfe der erfindungsgemäßen Mikroorganismen können prebiotische und insbesondere auch hefefreie Sauerteige bzw. Vorteige hergestellt werden, die Polyfructosane aufweisen, die für die besseren rheologische Eigenschaften der Teige und prebiotische Wirkung der Backware verantwortlich sind.

Ein weiterer Aspekt der Erfindung sind Polyfructosane enthaltende Sauerteige bzw. Vorteige in getrockneter, flüssiger oder pastöser Form, die als "Convenience Produkte" in der Backwarenindustrie für eine direkte Herstellungsweise von prebiotischen Backwaren eingesetzt werden können, d.h. der jeweilige Betrieb muß keinen betriebseigenen Sauerteig mit speziellen polyfructosanbildenden Kulturen führen (indirekte Führung). Aus wirtschaftlicher Sicht bleibt es dem Betrieb erspart, Zeit und Geld für die indirekte Herstellung von prebiotischen SauerteigenNorteigen aufwenden zu müssen.

Ferner betrifft die Erfindung auch ein Verfahren zur Herstellung eines Polyfructosans durch Fermentation von Bakterien der Art Lactobacillus sanfranciscensis DSMZ 10613 und Lactobacillus sanfranciscensis DSMZ 14373 in Einzelkultur oder Mischkultur. Als Nährmedium wird insbesondere das oben erwähnte erfindungsgemäße Nährmedium verwendet. Die von den Bakterien gebildeten extrazellulären Polyfructosane können mittels Zentrifuge isoliert und gewonnen werden.

Es ist auch möglich, dieses Verfahren in einem Sauerteig durchzuführen und anschließend die Exopolysaccharide aus dem Sauerteig zu gewinnen.

Die gewonnene Polyfructosane werden des weiteren als Isolat bezeichnet.

Das Isolat (Polyfructosane) kann in aufgereinigter Form als Zusatzstoff oder als Nahrungsergänzungsmittel in Lebensmitteln, insbesondere für Backwaren, eingesetzt werden. Als Nahrungsergänzungsmittel ist die bifidogene prebiotische Eigenschaft der Polyfructosane von besonderer Bedeutung; Als Zusatzstoffe sind sie geeignet, die rheologischen Eigenschaften von Lebensmittelzubereitungen positiv zu beeinflussen.

Die Isolate können konserviert, bevorzugt getrocknet, gefriergetrocknet oder schockgefriergetrocknet werden. Sie können als Pulver oder als Paste oder in flüssiger Form vorliegen. Diese Formen haben sich verpackungs- und transporttechnisch als besonders ökonomisch erwiesen.

Nachstehend wird die Erfindung anhand von Ausführungsbeispielen näher erläutert.

### Beispiel 1

### Künstliches Nährmedium

| | |
|---|---|
| Pepton aus Casein | 5 - 25 g/l |
| Hefe-Extrakt | 2 - 10 g/l |
| K₂HPO₄ x 3 H₂O | 0,5 - 5 g/l |
| K₂HPO₄ | 1 - 8 g/l |
| Cystein-HCL | 0,1 - 1,5 g/l |
| NH₄Cl | 0,5 - 8 g/l |
| Saccharose | 5 - 70 g/l |
| Fleischextrakt | 1 - 10 g/l |

Der pH-Wert wird vor dem Autoklavieren auf 6,2 eingestellt, anschließend wird das Medium bei 121 °C für 10 Min. autoklaviert. Nach dem Autoklavieren wird je 1 ml der folgenden Stammlösungen steril zugegeben:
A: MgSO₂ × 3 H₂O (50 - 200 g/l) separat autoklaviert
B: MnSO₄ × 4 H₂O (25 - 100g /l) separat autoklaviert
C: Vitaminmix sterilfiltriert bestehend aus:
   Biotin, Folsäure, Nikotinsäure, Pyridoxalphosphat, Thiamin,
   Riboflavin, Cobalamin, Panthothensäure je (0,1 - 1,0 g/l)

Das Impfgut (bei 30 °C) wird durch Zentrifugation geerntet, in physiologischer Kochsalzlösung resuspendiert und die gewaschene Zellmasse als Inokulat für die Sauerteig/Vorteige verwendet.

### Beispiel 2

Die Sauerteige bzw. Vorteige können nach verschiedensten Rezepturen hergestellt werden, beispielweise nach den folgenden zwei Rezepturen:
1. Roggenvollkornteig:

| | |
|---|---|
| Roggenvollkornmehl | 200 g |
| Wasser | 200 g |
| Saccharose | 20 g |
| Impfgut als Feuchtmasse | 10 g |

Teigtemperatur 30 °C, Stehzeit 24 - 48 Stunden
2. Weizensauerteig / Weizenvorteig

| | |
|---|---|
| Weizenmehl Typ 550 | 200 g |
| Wasser | 200 g |
| Saccharose | 20 g |
| Impfgut als Feuchtmasse | 10 g |

Teigtemperatur 25 °C, Stehzeit 24 - 48 Stunden.

## Patentansprüche

1. Verfahren zur Herstellung von prebiotischen Sauerteigen/Vorteigen aus mindestens einem Getreidemahlgut und Wasser unter Zusatz von mindestens einer Starterkultur, ggf. unter Weiterführung des Sauerteiges/Vorteiges unter fortlaufender Säuerung mittels Zugabe von Mehl und Wasser **dadurch gekennzeichnet, dass** das Anstellgut Exopolysaccharide bildende Mikroorganismen, bei denen es sich um Lactobacillus sanfranciscensis DSMZ 10613 oder DSMZ 14373 oder deren Mischung handelt, enthält, wobei es sich bei den Exopolysacchariden um extrazelluläre Polyfructosane handelt, und dass dem Getreidemahlgut Saccharose zugesetzt wird, wobei 0,2 - 30,0 Gew.%, bevorzugt 1,0 - 20,0 Gew.-%, besonders bevorzugt 5 - 15 Gew.-%, Saccharose bezogen auf das Mehl im Teig eingesetzt wird.

2. Verfahren nach Anspruch 1, **dadurch gekennzeichnet, dass** prebiotisch wirksame Substanzen in situ im SauerteigNorteig gebildet werden.

3. Lb. sanfranciscensis DSMZ 10613.

4. Lb. sanfranciscensis DSMZ 14373.

5. Verfahren zur Herstellung von Exopolysacchariden, insbesondere extrazellulären Polyfructosan, durch Fermentation von Bakterien der Gattung Lactobacillus, **dadurch gekennzeichnet, dass** Lb. sanfranciscensis DSMZ 10613 oder Lb. sanfranciscensis DSMZ 14373 oder deren Mischung eingesetzt werden, wobei ein Saccharose enthaltendes Nährmedium zur Anzucht verwandt wird.

6. Verfahren nach Anspruch 5, **dadurch gekennzeichnet, dass** das Saccharose enthaltendes Nährmedium Pepton, Hefeextrakt, Fleischextrakt, Dikaliumhydrogenphosphat, Kaliumdihydrogenphosphat, Cystein-HCI, Ammoniumchlorid, Saccharose und eine Stammlösung aufweist.

7. Verfahren nach Anspruch 6, **dadurch gekennzeichnet, dass** die Stammlösung Magnesiumsulfat, Mangansulfat und einen Vitaminmix enthält.

8. Verfahren nach Anspruch 7, **dadurch gekennzeichnet, dass** der Vitaminmix Biotin, Folsäure, Nikotinsäure, Pyridoxalphosphat, Thiamin, Riboflavin, Cobalamin, Panthothensäure aufweist.

9. Verfahren nach Anspruch 5, **dadurch gekennzeichnet, dass** es in einem Sauerteig durchgeführt wird.

## Claims

1. A method of producing prebiotic sourdoughs/predoughs from at least one ground grain material and water with the addition of at least one starter culture, optionally with propagation of the sourdough/predough with continuing acidification by means of the addition of flour and water, **characterised in that** the starting material contains microorganisms which form exopolysaccharides, which are Lactobacillus sanfranciscensis DSMZ 10613 or DSMZ 14373 or a mixture thereof, whereby the exopolysaccharides are extracellular polyfructosanes, and that saccharose is added to the ground grain material, whereby 0.2-30.0 wt.%, preferably 1.0-20.1 wt.%, particularly preferably 5-15 wt.% saccharose with respect to the flour is used in the dough.

2. A method as claimed in claim 1, **characterised in that** pre-biotically active substances are formed in situ in the sourdough/predough.

3. Lb sanfranciscensis DSMZ 10613.

4. Lb sanfranciscensis DSMZ 14373.

5. A method of producing exopolysaccharides, particular extracellular polyfructosane, by fermentation of bacteria of the type Lactobacillus, **characterised in that** Lb sanfranciscensis DSMZ 10613 or Lb sanfranciscensis DSMZ 14373 or a mixture thereof is used, whereby a nutrient medium containing saccharose is used for the production.

6. A method as claimed in claim 5, **characterised in that** the nutrient medium containing saccharose includes peptone, yeast extract, dipotassium phosphate, monopotassium phosphate, cysteine-HCl, ammonium chloride, saccharose and a stock solution.

7. A method as claimed in claim 6, **characterised in that** the stock solution contains magnesium sulphate, manganese sulphate and a vitamin mix.

8. A method as claimed in claim 7, **characterised in that** the vitamin mix includes biotin, folic acid, nicotinic acid, pyridoxal phosphate, thiamine, riboflavine, cobalamine and panthothenic acid.

9. A method as claimed in claim 5, **characterised in that** it is conducted in a sourdough.

## Revendications

1. Procédé pour la préparation de prélevains/levains de panification prébiotiques constitués d'au moins une mouture de céréales et d'eau avec ajout d'au moins une culture de démarrage, éventuellement avec traitement du prélevain/levain de panification avec acidification continue par ajout de farine et d'eau, **caractérisé en ce que** la préparation contient des microorganismes formant des exopolysaccharides qui sont Lactobacillus sanfranciscensis DSMZ 10613 ou DSMZ 14373 ou leur mélange, les exopolysaccharides étant des polyfructosanes extracellulaires, et **en ce qu'**à la mouture de céréales est ajouté du saccharose, en utilisant 0,2-30,0 % en poids, de préférence 1,0-20,0 % en poids, de plus grande préférence 5-15 % en poids de saccharose par rapport à la farine contenue dans la pâte.

2. Procédé selon la revendication 1, **caractérisé en ce que** des substances à activité prébiotique sont formées in situ dans le prélevain/levain de panification.

3. Lb. sanfranciscensis DSMZ 10613.

4. Lb. sanfranciscensis DSMZ 14373.

5. Procédé de fabrication d'exopolysaccharides, en particulier de polyfructosane extracellulaire, par fermentation de bactéries du genre Lactobacillus, **caractérisé en ce que** l'on utilise Lb. sanfranciscensis DSMZ 10613 ou Lb. sanfranciscensis DSMZ 14373 ou leur mélange, un milieu nutritif contenant du saccharose étant utilisé pour la culture.

6. Procédé selon la revendication 5, **caractérisé en ce que** le milieu nutritif contenant du saccharose présente du peptone, un extrait de levure, un extrait de viande, de l'hydrogène diphosphate de potassium, du dihydrogène phosphate de potassium, cystéine-HCl, du chlorure d'ammonium, du saccharose ou une solution souche.

7. Procédé selon la revendication 6, **caractérisé en ce que** la solution souche contient du sulfate de magnésium, du sulfate de manganèse ou un mélange de vitamines.

8. Procédé selon la revendication 7, **caractérisé en ce que** le mélange de vitamines contient la biotine, l'acide folique, l'acide nicotinique, le phosphate de pyridoxal, la thiamine, la riboflavine, la cobalamine, l'acide pantothénique.

9. Procédé selon la revendication 5, **caractérisé en ce qu'**il est réalisé dans un levain.
